# FASCICULE DE BREVET EUROPEEN

(11) **EP 0 686 601 B1**
(45) Date de publication et mention de la délivrance du brevet: **20.01.1999**
(21) Numéro de dépôt: 95106809.7
(22) Date de dépôt: 05.05.1995
(51) Int. Cl.: C02F 1/32, A61L 2/10, A23L 3/28, B01J 19/12

(54) **Procédé et dispositif de traitement des liquides par rayonnement ultraviolet**
Verfahren und Gerät zur Flüssigkeitsbehandlung durch UV
Process and device for treating liquids with UV radiation

(30) Priorité: 09.05.1994 FR 9406002
(43) Date de publication de la demande: 13.12.1995
(73) Titulaire: De Stoutz, Jean-Christian, 74500 Evian-Les-Bains (FR)
(72) Inventeur: De Stoutz, Jean-Christian, 74500 Evian-Les-Bains (FR)
(74) Mandataire: Micheli, Michel-Pierre

(56) Documents cités:
- US-A- 4 028 246

## Description

La présente invention concerne un procédé de traitement des liquides en vue de leur désinfection par rayonnement ultraviolet ainsi qu'un dispositif pour la mise en oeuvre de ce procédé. On connaît depuis longtemps le principe de désinfection des liquides, en particulier de l'eau de consommation, par irradiation au moyen d'un rayonnement ultraviolet. Ce traitement présente de nombreux avantages par rapport aux méthodes chimiques classiques de traitement des eaux, en particulier l'absence d'odeur et de goût désagréable dans l'eau traité. On évite également le développement de sous-produits nocifs telles que les aliformes et les choramines dans le cas d'un traitement au chlore.

Les effets germicides de la radiation solaire sont connus depuis longtemps et, plus particulièrement, les ultraviolets UVC, dont la longueur d'onde se situe entre 200 et 280 nm., ont une action germicide en provoquant l'arrêt des fonctions de reproduction des micro-organismes par modification de leur structure moléculaire interne.

L'efficacité germicide des dispositifs utilisant ce principe est fonction de différents facteurs essentiels comme la puissance de la source émettrice d'ultraviolet (UV), le temps d'exposition et le coefficient d'absorption du liquide traité.

D'autres facteurs comme la turbidité ou la couleur du liquide, les matières en suspension dans le liquide et la distance entre le germe et la source d'UV sont également déterminants pour garantir une désinfection de bonne qualité. Les dispositifs existants de traitement de liquide par irradiation ultraviolet comportent généralement une chambre de traitement comprenant les générateurs UV dans laquelle circule le liquide à traiter. Cette chambre de traitement est réalisée dans une matière assurant une bonne réflexion des rayons ultraviolets comme de l'acier inoxydable par exemple. Le faible pouvoir de pénétration des rayons ultraviolets dans les liquides faiblement ou fortement opaques nécessite un écoulement turbulent du fluide à travers la chambre d'irradiation, de sorte que le fluide soit irradié de manière homogène.

On connaît encore du document US-A-4028246 un procédé et un dispositif de traitement de l'eau dans lequel l'eau est dirigée vers des surfaces où l'eau forme un film s'écoulant par gravité le long de parois verticales. Ce film d'eau est soumis à un traitement par ozone et éventuellement irradié par des sources de rayons ultraviolets.

Ces dispositifs présentent l'inconvénient d'être complexes et coûteux à concevoir et à fabriquer. De plus, les dépôts laissés sur les sources émettrices UV par les liquides chargés en matière organique, ou par les produits visqueux par effet d'adhérence, nécessitent que l'on procède régulièrement à un nettoyage mécanique ou chimique de ces dispositifs.

Enfin, l'écoulement du liquide par gravité est généralement lent, ce qui nécessite de grandes surfaces pour pouvoir traiter de grands débits d'eau.

Le but de la présente invention est d'obvier aux inconvénients cités ci-dessus. La titulaire propose à cet effet un procédé ainsi qu'un dispositif de traitement des liquides par rayonnement ultraviolet qui se distinguent par les caractéristiques énumérées aux revendications 1 et 3.

Le dessin annexé illustre schématiquement et à titre d'exemple une forme d'exécution du dispositif de traitement selon la présente invention.

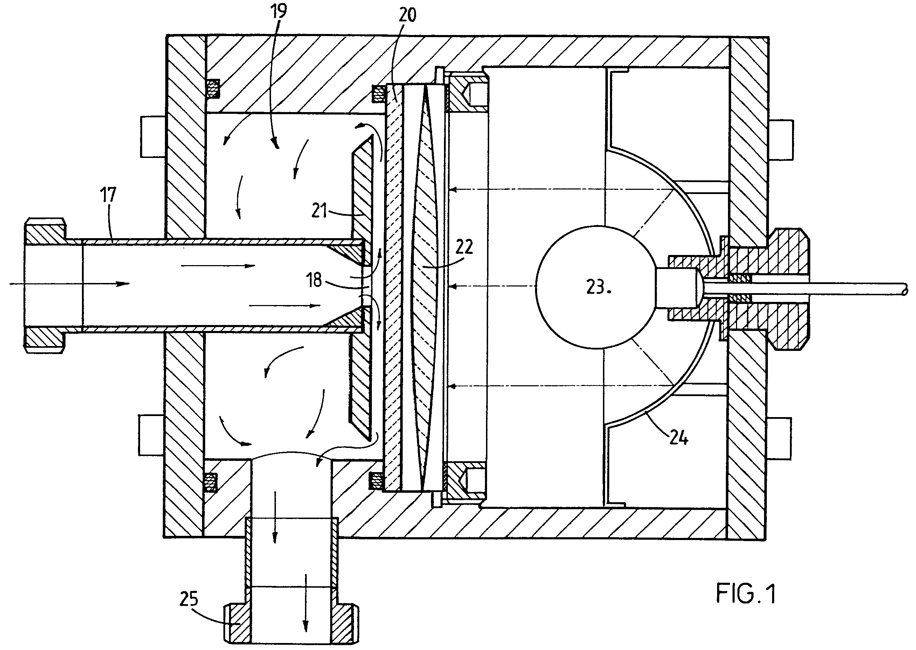

La figure 1 est une vue en coupe d'une forme d'exécution du dispositif de traitement.

L'exécution du dispositif illustré a la figure 1 comporte une tuyère d'admission 17 alimentée sous pression avec le liquide à traiter. Cette tuyère 17 comporte un orifice de sortie 18 qui débouche dans une chambre de traitement 19. Le fond de cette chambre 19 est constitué par une paroi cible 20 réalisée en quartz ou en verre-céramique, ou toute autre matière présentant une bonne perméabilité au rayonnement ultraviolet. Une plaque 21 est fixée sur la tuyère d'admission 17 et s'étend parallèlement à la paroi cible 20. Le liquide projeté contre la cible 20 forme un film entre ladite paroi 20 et la plaque 21. Une lentille 22 située derrière la paroi cible 20 permet de concentrer le rayonnement généré par une lampe à ultraviolet 23 munie d'un déflecteur 24. La source d'ultraviolet 23 est complètement isolée de la chambre de traitement 19 dans laquelle circule le liquide, ce qui évite tout contact préjudiciable entre le liquide et la source émettrice. En ajustant la force de projection du liquide sur la cible 20 on assure un autonettoyage de cette dernière et de ce fait un fonctionnement en continu. La chambre de traitement 19 est encore pourvue d'un conduit de récupération du liquide dans sa partie inférieure. Le dispositif fonctionne de la façon suivante : le liquide injecté sous pression dans la tuyère d'admission 17 est projeté contre la paroi cible 20. Il se forme entre la plaque de guidage 19 et la paroi cible 20 un film de liquide dont l'épaisseur est fonction de la vitesse de projection du liquide. Ce film de liquide est irradié par la source émettrice d'ultraviolets 23 dont on peut concentrer le rayonnement au centre la paroi 20 grâce à la lentille convergente 22. Le liquide, une fois irradié, est récupéré dans le fond de la chambre de traitement et évacué par le conduit de refoulement 25.

Le dispositif décrit est un exemple qui permet la mise en oeuvre d'un procédé de traitement des liquides par ultraviolet. La première étape de ce procédé consiste à former un film de liquide sur une surface cible par projection de liquide contre celle-ci, dont on peut faire varier l'épaisseur. Plus le liquide à traiter est opaque, ou chargé de micro-particules en suspension, plus le film à former sera mince afin que la pénétration des ultraviolets soit suffisante pour assurer le traitement du liquide. La deuxième étape du procédé consiste à soumettre le film de liquide ainsi obtenu à un rayonnement ultraviolet. Dans le cas d'un écoulement non laminaire du film de liquide comme la forme d'exécution décrite, on ajuste l'épaisseur du film liquide. L'irradiation par la source émettrice d'ultraviolet se fait en transparence à travers une matière perméable au rayonnement ultraviolet. Les rayons peuvent également être concentrés grâce à un jeu de lentilles convergentes. La dernière étape du procédé consiste en la récupération du liquide traité. Ce procédé offre l'avantage par rapport aux procédés existants de pouvoir adapter facilement et de façon déterminée les paramètres (temps d'irradiation, épaisseur du film) nécessaires à une désinfection de bonne qualité. Contrairement aux procédés existants dans lesquels il faut s'assurer grâce à des configurations complexes des chambres de traitement, ainsi qu'à l'aide d'instruments de contrôle sophistiqués et coûteux, que le liquide a été suffisamment irradié en chaque point, le procédé, objet de la présente invention ne nécessite aucun appareillage complexe pour sa mise en oeuvre et il est facilement paramétrable en fonction de la turbidité ou de l'opacité des liquides à traiter.

## Revendications

1. Procédé de traitement d'un liquide par rayonnement ultraviolet, caractérisé par le fait qu'on forme un film de liquide sur une surface cible transparente par projection du liquide sur cette surface; qu'on ajuste en fonction des caractéristiques du liquide à traiter l'épaisseur du film sur la surface cible; qu'on soumet le film liquide à un rayonnement ultraviolets au travers de la surface cible transparente; puis qu'on récupère le liquide traité.

2. Procédé selon la revendication 1, caractérisé par le fait que l'on projette le liquide à traiter à l'aide d'une buse au centre d'une surface cible circulaire.

3. Dispositif pour le traitement d'un liquide par rayonnement ultraviolet comportant une chambre de traitement (19) du liquide présentant un conduit d'admission (17) et un conduit d'évacuation (25) du liquide, caractérisé par le fait qu'une paroi terminale (20) de cette chambre (19) est réalisée en un matériau transparent et constitue une surface cible par le fait que le conduit d'admission (17) traverse la chambre de traitement (19) sensiblement perpendiculaire à cette face terminale transparente (20) et se termine à proximité de celle-ci par une buse projetant le liquide à traiter contre la zone centrale de cette paroi terminale (20); par le fait qu'il comporte encore de l'autre côté de la paroi terminale (20) transparente une source de rayons ultraviolets (23).

4. Dispositif selon la revendication 3, caractérisé par le fait que le conduit d'admission (17) est solidaire d'un disque (21) disposé dans un plan parallèle à la paroi transparente (10) de la chambre (19) forçant ainsi le liquide à former un film entre ce disque (21) et cette paroi (20).

5. Dispositif selon la revendication 3 ou la revendication 4, caractérisé par le fait qu'une lentille (22) est située entre la source de rayon UV (23) et la paroi transparente (20).

## Claims

1. Method for the treatment of a liquid through ultraviolet radiation, characterised by the fact that a liquid film is formed on a transparent target surface by projection of said liquid against said surface; the thickness of the liquid film on the target surface is adjusted in function of the characteristics of the liquid to be treated; and the liquid film is submitted to ultraviolet radiation through the transparent target surface; and the treated liquid is then recuperated.

2. Method according to claim 1, characterised by the fact that the liquid to be treated is projected through a nozzel onto the center of a circular target surface.

3. Device for the treatment of a liquid by ultraviolet radiation comprising a treatment chamber (19) of the liquid presenting an admission duct (17) and a exhaust duct (25) of the liquid, characterised by the fact that a end wall (20) of that chamber (19) is made in a transparent material and constitute a target surface; by the fact that the admission duct (17) crosses the treatment chamber (19) substantially perpendicularly to said transparent end wall (20) and is terminated in its vicinity by a nozzel projecting the liquid to be treated against the central zone of said end wall (20); by the fact that it comprises further on the other side of the transparent end wall (20) a source of ultraviolet rays (23).

4. Device according to claim 3, characterized by the fact that the admission duct (17) is fastened with a disc (21) located in a plan parallel to the transparent wall (20) of the chamber (19) thereby forcing the liquid to form a film between this disc (21) and the wall (20).

5. Device according to claim 3 or 4, characterized by the fact that a lense (22) is located between the UV ray source (23) and the transparent wall (20).

## Patentansprüche

1. Verfahren zur Behandlung einer Flüssigkeit durch ultraviolette Strahlung, dadurch gekennzeichnet, dass man auf einer durchsichtigen Auffangfläche durch Aufspritzen der Flüssigkeit auf diese Fläche einen Flüssigkeitsfilm bildet; dass man in Abhängigkeit von den Eigenschaften der zu behandelnden Flüssigkeit die Dicke des Films auf der Auffangfläche anpasst; dass man den Flüssigkeitsfilm durch die durchsichtige Auffangfläche hindurch einer ultravioletten Strahlung aussetzt; und dass man dann die behandelte Flüssigkeit zurückgewinnt.

2. Verfahren gemäss Anspruch 1, dadurch gekennzeichnet, dass man die zu behandelnde Flüssigkeit mit Hilfe einer Düse auf die Mitte einer kreisrunden Auffangfläche aufspritzt.

3. Vorrichtung zur Behandlung einer Flüssigkeit durch ultraviolette Strahlung mit einer Kammer (19) für die Behandlung der Flüssigkeit, die eine Zuleitung (17) und eine Ableitung (25) für die Flüssigkeit aufweist, dadurch gekennzeichnet, dass eine endständige Wandung (20) dieser Kammer (19) aus einem durchsichtigen Werkstoff gefertigt ist und eine Auffangfläche darstellt; dadurch, dass die Zuleitung (17) die Behandlungskammer (19) im wesentlichen im rechten Winkel zu dieser durchsichtigen endständigen Fläche (20) quert und nahe dieser Fläche in eine Düse ausläuft, die die zu behandelnde Flüssigkeit auf den mittleren Bereich dieser endständigen Wandung (20) aufspritzt; und dadurch, dass sie noch auf der anderen Seite der endständigen, durchsichtigen Wandung (20) eine Quelle ultravioletter Strahlen (23) umfasst.

4. Vorrichtung gemäss Anspruch 3, dadurch gekennzeichnet, dass die Zuleitung (17) fest mit einer Scheibe (21) verbunden ist, die in einer Ebene angeordnet ist, die parallel zur durchsichtigen Wandung (20) der Kammer (19) verläuft und somit die Flüssigkeit zwingt, einen Film zwischen dieser Scheibe (21) und dieser Wandung (20) auszubilden.

5. Vorrichtung gemäss Anspruch 3 oder 4, dadurch gekennzeichnet, dass eine Linse (22) zwischen der Quelle von UV-Strahlen (23) und der durchsichtigen Wandung (20) angeordnet ist.
